# EUROPEAN PATENT SPECIFICATION

(11) **EP 3 719 011 B1**
(45) Date of publication and mention of the grant of the patent: **29.03.2023**
(21) Application number: 18896218.7
(22) Date of filing: 21.12.2018
(51) Int. Cl.: C07D 401/04, C07C 237/36, C07C 231/14, A01N 43/56

(54) **PYRAZOLE AMIDE COMPOUND HAVING PESTICIDAL ACTIVITY AND USE THEREOF**
PYRAZOLAMIDVERBINDUNG MIT PESTIZIDER AKTIVITÄT UND VERWENDUNG DAVON
COMPOSÉ PYRAZOLE AMIDE PRÉSENTANT UNE ACTIVITÉ PESTICIDE ET SON UTILISATION

(30) Priority: 29.12.2017 CN 201711498597; 07.12.2018 CN 201811502022
(43) Date of publication of application: 07.10.2020
(73) Proprietor: Jiangsu Flag Chemical Industry Co., Ltd., Nanjing, Jiangsu 210047 (CN)
(72) Inventor: FENG, Meili, Nanjing, Jiangsu 210047 (CN); LI, Hongju, Nanjing, Jiangsu 210047 (CN); SHI, Xinxin, Nanjing, Jiangsu 210047 (CN)
(74) Representative: Laufhütte, Dieter
(86) International application number: PCT/CN2018/122724
(87) International publication number: WO 2019/128872

(56) References cited:
- EP-A1- 3 075 729
- WO-A1-2016/070562
- CN-A- 1 653 051
- CN-A- 104 447 688
- CN-A- 104 447 688
- CN-A- 107 056 747
- US-B2- 8 217 179

## Description

### BACKGROUND

### Technical Field

The present invention relates to the technical field of insecticides, and particularly to a pyrazolamide compound having an insecticidal activity and use thereof.

### Related Art

Development of resistance to insecticides of pests is often a potential puzzling problem. This is also one of the most important reasons why pesticide researchers have been seeking to develop insecticides with special mechanisms of action. Over the years, pesticide researchers have been working hard to find pesticides with special mechanisms of action. The o-formamido-benzamide compounds developed by DuPont are a new class of compounds that target ryanodine receptor. The representative compound chlorantraniliprole (RynaxypyrTM) shows excellent comprehensive insecticidal activity and field effects, low toxicity to mammals, and good environmental compatibility.

The patent CN 104447688 reports 2-benzamidopropionamide compounds, of which the compounds KC1 and KC2 having structural formulas shown below have good control effects on *Plutella xylostella, Mythimna separata* and aphids.

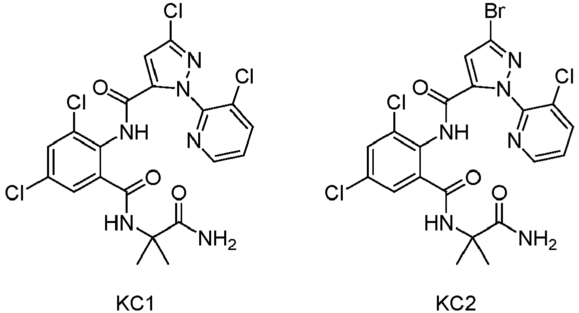

In the prior art, the benzamide compound and insecticidal activity thereof shown in the present invention has not been disclosed.

CN 107 056 747 discloses an amide derivative containing an alpha-aminoketone structure and a preparation method and application thereof. US 8 217 179 belongs to the field of organic synthesis, and specifically relates to the preparation method of phenylcarboxamides.

### SUMMARY

The present invention provides a benzamide compound with novel structure and higher insecticidal effect, which is useful in the control of pests.

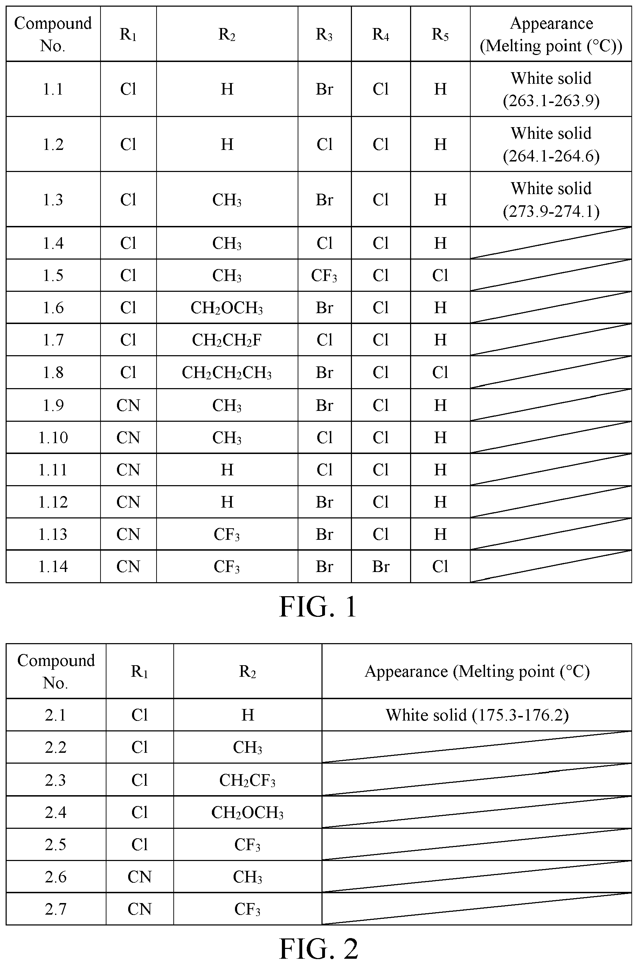

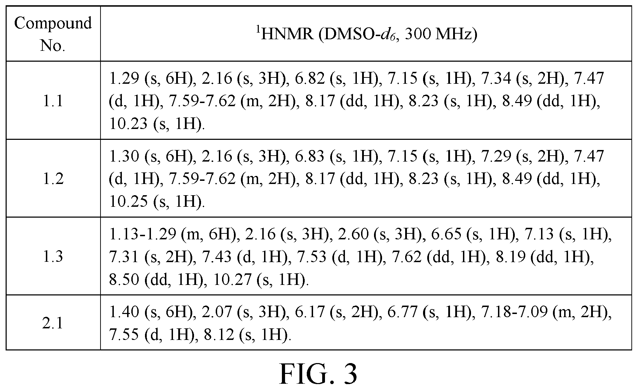

The following technical solutions are adopted in the present invention. A benzamide compound of General Formula I is provided: 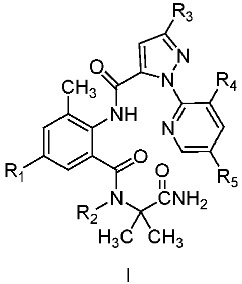 In General Formula I:
R₁ is selected from chloro or CN; R₂ is selected from H, C₁-C₃ alkyl, C₁-C₃ haloalkyl or C₁-C₅ alkoxyalkyl; R₃ is selected from halo or C₁-C₃ haloalkyl; R₄ is selected from halo; and R₅ is selected from H or halo. The physical properties of some compounds of General Formula I are shown in Fig. 1. Some compounds of General Formula I are tested by ¹HNMR spectroscopy. The results of ¹HNMR spectroscopy (DMSO-d₆, 300 MHz) are shown in Fig. 3.
The preferred compounds in present invention are those of General Formula I in which
R₁ is selected from chloro or CN; R₂ is selected from H, C₁-C₃ alkyl, CH₂OCH₃, CH₂OCH₂CH₃, CH₂CH₂OCH₃, CF₃, CH₂CH₂F, CH₂CHF₂, CH₂CF₃, CF₂CF₃ or CF(CF₃)₂; R₃ is selected from chloro, bromo or trifluoromethyl; R₄ is selected from chloro; and R₅ is selected from H or chloro.

More preferred compounds are those of General Formula I in which R₁ is selected from chloro; R₆ is H or methyl; R₃ is selected from chloro or bromo; R₄ is selected from chloro; and R₅ is selected from H.

The present invention also involves an intermediate useful in the direct preparation of the compound of General Formula I. This intermediate has not been reported previously. The intermediate has a structure of General Formula II: 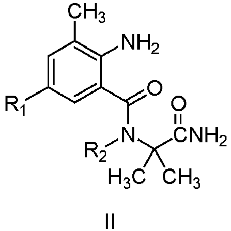 In General Formula II:
R₁ is selected from halo or CN; R₂ is selected from H, C₁-C₃ alkyl, C₁-C₃ haloalkyl or C₁-C₅ alkoxyalkyl. The physical properties of some compounds of General Formula II are shown in Fig. 2.

Hereinafter, a typical preparation method of the present invention is shown, but it is not intended to limit the scope of the present invention in any way.

The compound of General Formula I can be prepared by Reaction Scheme 1, where the substituents are as defined above unless otherwise specified.

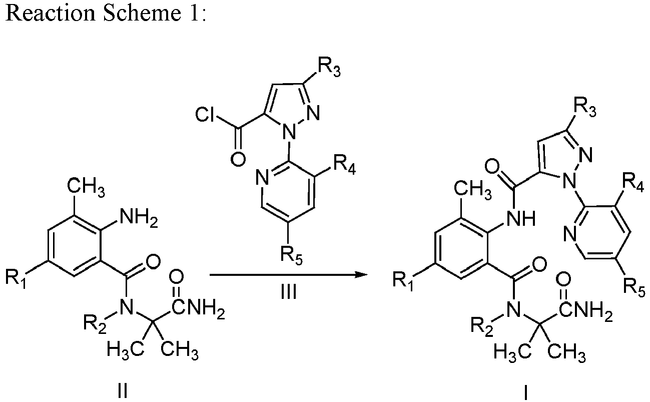

In a suitable solvent, a compound of General Formula II is reacted with a compound of General Formula III in the presence or absence of a base to obtain a compound of General Formula I.

The addition of an appropriate amount of a base is beneficial to the reaction. Useful organic bases include, for example, pyridine, triethylamine, potassium tert-butoxide, 4-dimethylaminopyridine or N-methylmorpholine. Useful inorganic bases include, for example, sodium hydride, sodium bicarbonate, sodium carbonate, potassium carbonate, and sodium hydroxide. The reaction is carried out in a suitable inert solvent such as tetrahydrofuran, acetonitrile, toluene, dichloromethane, and the like.

After the reaction is completed, the reaction mixture containing the intended product is separated following a common method, and if necessary, purified by recrystallization or column chromatography, thereby obtaining the intended product. These methods are well documented in literatures, for example, J. Org. Chem. 32, 3069 (1967).

The compound of General Formula II can be prepared by Reaction Scheme 2, where the substituents are as defined above unless otherwise specified.

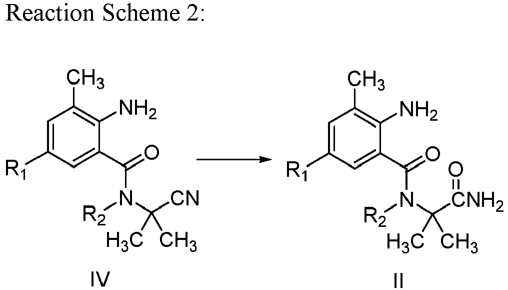

A compound of General Formula IV is reacted with a hydrogen peroxide solution in the presence of a base, to obtain a compound of General Formula III. The base is selected from sodium hydroxide, or potassium hydroxide, etc. The reaction is carried out in a suitable inert solvent such as tetrahydrofuran, acetonitrile, toluene, dichloromethane, and the like. After the reaction is completed, the reaction mixture containing the intended product is separated following a common method, and if necessary, purified by recrystallization or column chromatography, thereby obtaining the intended product.

The compound of General Formula IV can also be prepared by Reaction Scheme 3, where the substituents are as defined above unless otherwise specified.

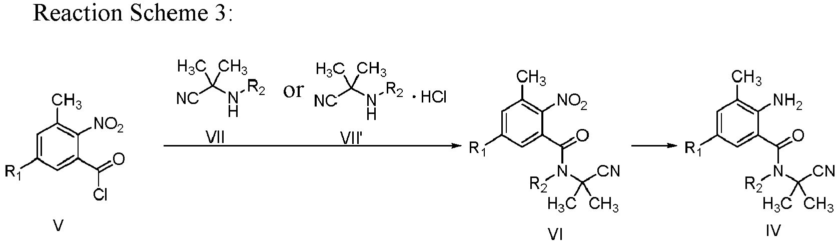

The compound of General Formula V can be prepared by a known general method (for example, Organic Syntheses, 9, 32 (1929)). The compound of General Formula Vis easily prepared with a commercially available chloroformylating reagent such as chlorosulfoxide, and oxalyl chloride.

Some of the compounds of General Formula VII or VII' are commercially available, and some are prepared by a known general method, for example, as described in J. Am. Chem. Soc., 75, 4841-4842(1953), and Chemical Communications, 48(50), 6253-6255(2012).

### (1) General Formula V→General Formula VI

The compound of General Formula V is reacted with the compound of General Formula IV to obtain the compound of General Formula VI. A known method, for example, as described in J. Am. Chem. Soc., 135(12), 4628-4631(2013), can be used.

### (2) General Formula VI→General Formula II

A typical method includes hydrogenation reduction in a hydroxylic solvent such as ethanol, methanol, and isopropanol in the presence of a metal catalyst such as Pd/C, platinum oxide or Ni (for example, Chinese Journal of Chemical Engineering, 24(9), 1195-1200 (2016)). It can also be prepared by reduction with metals such as zinc powder and iron powder in the presence of an acid catalyst. These methods are generally described in literatures, such as WO 2010042699; and Dye Industry, 37(4): 16-18(2000).

In an organic molecule, the substitution of hydrogen or halogen atom(s) with an electron donating methyl group or other alkyl groups can alter the liposolubility solubility of the molecule. It can be known from the analysis of nuclear magnetic data that the introduction of methyl in the present invention has caused changes in the spatial arrangement of molecules. The liposolubility of a molecule is closely related to the conduction of the molecule in plants, insects, and other organisms. Changes in the spatial structure of a molecule also affect the ability of the molecule to bind to the target. These two factors play an important role in the effectiveness of an agent. The effects of the liposolubility and the changes in spatial structure of a molecule on the conductivity and the ability of the bioactive molecule to bind to a target are unpredictable and can be known only after a lot of creative efforts.

It has been found that compared with known benzamide compounds, the compound of General Formula I of the present invention has unexpectedly high insecticidal activity. Therefore, the present invention also involves use of the compound of General Formula I in controlling pests.

The present invention also involves an insecticidal composition having the compound of General Formula I as an active ingredient. The content in percentage by weight of the active ingredient in the insecticidal composition is between 1-99%. The insecticidal composition also comprises an agriculturally, forestrically, and hygienically acceptable carrier.

The composition of the present invention can be applied in the form of a formulation. The compound of General Formula I, as an active ingredient, can be dissolved or dispersed in a carrier or formulated into a formulation for easier dispersion when used as an insecticide. For example, these chemicals can be made into wettable powders or emulsifiable concentrates. In these compositions, at least one liquid or solid carrier is added, and an appropriate surfactant can be added when needed.

The technical solutions of the present invention also include a method for controlling pests by applying a insecticidal composition of the present invention to the pests or their growth media excluding the human and animal body. Generally, a more suitable effective amount is 10 to 1000 g per hectare.

For some applications, for example, in agriculture, one or more other fungicides, insecticides, herbicides, plant growth regulators or fertilizers may be added to the insecticidal composition of the present invention, thereby bringing about additional advantages and effects.

The present invention has the following advantages. The present invention discloses two types of compounds. Compared with the compounds in the prior art, the compound of General Formula I has a higher insecticidal activity against pests such as *Spodoptera litura, Jacobiasca formosana, Frankliniella intonsa, Nilaparvata lugens,* and *Helicoverpa armigera,* and has a good insecticidal effect even at a concentration of below 40 ppm. Compared with similar compounds in the prior art, the amount of the compound used and the residue of the compound in farmland are reduced, and thus the compound of the present invention is environmentally friendly.

The compound of General Formula II is an intermediate for synthesizing the compound of General Formula I. The method of the present invention for synthesizing the compounds of General Formula I and General Formula II solves the problem of inconvenient synthesis of similar compounds in the prior art, is more suitable for industrial applications, and reduces the production cost of manufacturers.

### BRIEF DESCRIPTION OF THE DRAWINGS

Fig. 1 shows the physical properties of some compounds of General Formula I according to the present invention.
Fig. 2 shows the physical properties of some compounds of General Formula II according to the present invention.
Fig. 3 shows the test results of some compounds of General Formula I by ¹HNMR spectroscopy.

### DETAILED DESCRIPTION

The present invention is further described in detail below with reference to specific embodiments, but the embodiments are not intended to limit the present invention.

Unless otherwise stated, the following terms used in the specification and claims have the meanings discussed below. "Alkyl" means a saturated aliphatic hydrocarbon group, including linear and branched forms, such as methyl, ethyl, propyl, isopropyl, and the like. "Haloalkyl" means a group in which an alkyl group is substituted with one or more halogen atoms, such as chloroethyl, trifluoromethyl, and the like. "Alkoxyalkyl" means a group with an oxygen atom attached to the end of an alkyl group, such as methoxymethyl, methoxyethyl, ethoxymethyl and the like.

### Example 1

### Synthesis of Compound 1.1: N-(2-N-((1-amino-2-methyl-1-acylpropan-2-yl)-formyl)-4-chloro-6-methylphenyl)-1-(3 -chloropyridin-2-yl)-3 -bromo -1H-pyrazol-5-carboxamide

### (1) Synthesis of 2-nitro-3-methylbenzoyl chloride

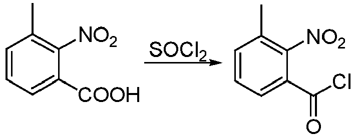

2-nitro-3-methylbenzoic acid (10.0 g, 0.055 mol), dichloroethane (200 ml), chlorosulfoxide (23.8 g, 0.2 mol), and DMF (1 drop) were sequentially added to a 250 ml single-necked flask, heated to reflux and reacted for 3 h. Then, the solvent was removed under elevated pressure to obtain a brown liquid (11.0 g, yield 100%). The product was directly used in next step without further post-treatment.

### (2) Synthesis of N-(1-cyanoisopropyl) -3-methyl-2-nitrobenzamide

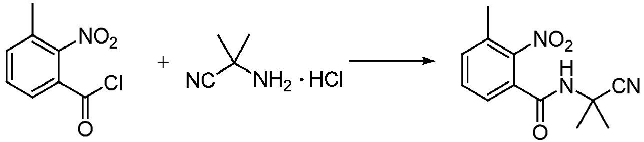

2-methyl-2-(methylamino)propionitrile hydrochloride (6.0 g, 0.05 mol), tetrahydrofuran (20 ml), water (20 ml), and NaHCOs (8.4 g, 0.1 mol) were sequentially added to a 250 ml four-necked flask. 2-nitro-3-methylbenzoyl chloride (10 g, 0.05 mol) in tetrahydrofuran (20 ml) was added dropwise at -10°C, and then continuously reacted for 2 h with stirring at -10°C. The reaction solution was added with water (50 ml), and extracted with ethyl acetate (20 ml x 3). The organic phases were combined, washed with saturated aqueous sodium chloride solution, and dried over anhydrous sodium sulfate. The solvent was removed under reduced pressure to obtain a brown solid (6.0 g, yield 48.2%).

### (3) Synthesis of N-(1-cyanoisopropyl) -3-methyl-2-aminobenzamide

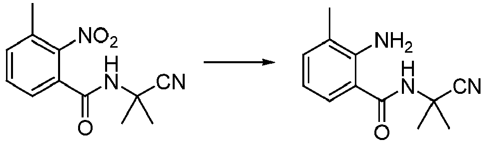

Water (20 ml), reduced Fe powder (2.2 g, 0.04 mol), and 30% hydrochloric acid (1 ml) were sequentially added to a 100 ml four-necked flask, slowly heated to 80°C and stirred for 30 min at 80°C. Then, N-(1-cyanoisopropyl)-3-methyl-2-nitrobenzamide (2.5 g, 0.01 mol) was added batchwise, while the temperature was maintained at no more than 80°C. After that, the reaction was continuously stirred at 80°C, until the reaction was completed as indicated by HPLC. The reaction solution was cooled to room temperature, added with sodium hydroxide (1.6 g), and filtered with suction. The filter cake was washed with hot water, and the collected filtrate was extracted with ethyl acetate (2 x 100 ml). The organic phase was washed with water, saturated sodium carbonate solution and saturated brine, and dried over anhydrous sodium sulfate. The solvent was removed under reduced pressure to obtain a brown solid (1.4 g, yield 63.1%).

### (4) Synthesis of N-(1-cyanoisopropyl)-2-amino-3-methyl-5-chlorobenzamide

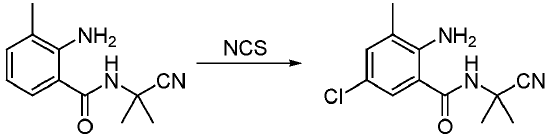

N-(1-cyanoisopropyl)-3-methyl-2-aminobenzamide (1.5 g, 6.9 mmol), N-chlorosuccinimide (1.4 g, 10.3 mmol) and DMF (20 ml) were sequentially added to a 100 ml four-necked flask, and reacted with stirring at room temperature until the reaction was completed as indicated by HPLC. The reaction solution was poured into water (100 ml), and extracted with ethyl acetate (20 ml × 3). The organic phases were combined, washed sequentially with saturated aqueous sodium chloride solution and water, and dried over anhydrous sodium sulfate. The solvent was removed under reduced pressure. The resulting crude product was purified by column chromatography (petroleum ether: ethyl acetate = 5:1) to obtain a white solid (1.4 g, yield 80.1%).

### (5) Synthesis of N-(1-amino-2-methyl-1-acylpropan-2-yl)-2-amino-3-methyl-5-chlorobenzamide

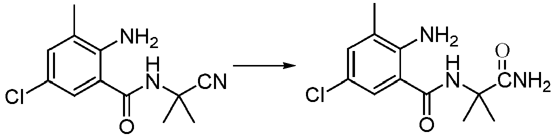

In an ice bath, N-(1-cyanoisopropyl)-2-amino-3-methyl-5-chlorobenzamide (1 g, 4.0 mmol), tetrahydrofuran (10 ml), sodium hydroxide (0.24 g, 6.0 mmol) in water (5 ml), and 30% hydrogen peroxide solution (2 g, 18 mmol) were sequentially added to a 100 ml single-necked flask, naturally warmed to room temperature, and reacted with stirring, until the reaction was completed as indicated by HPLC. The solvent was removed from the reaction solution under reduced pressure to obtain a brownish-yellow solid. The solid was washed with tetrahydrofuran (10 ml × 2), and filtered. The filtrate was collected, and removed of the solvent under reduced pressure. The crude product was purified column chromatography (petroleum ether: ethyl acetate = 1:1) to obtain a white solid (0.39 g, yield 40.1%).

### (6) Synthesis of Compound 1.1: N-(2-N-((1-amino-2-methyl-1-acylpropan-2-yl)-formyl)-4-chloro-6-methylphenyl)-1-(3 -chloropyridin-2-yl)-3 -bromo -1H-pyrazol-5-carboxamide

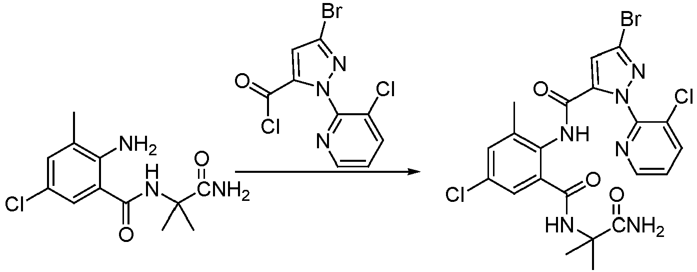

N-(1-amino-2-methyl-1-acylpropan-2-yl)-N-methyl-2-amino-3 -methyl-5-chlorob enzamide (0.27 g, 1 mmol), acetonitrile (10 ml), pyridine (0.1 g, 1 mmol), and 2-(3-chloro-pyridin-2-yl)-5-bromo -2H-pyrazol-3-carbonyl chloride (0.32 g, 1 mmol) (prepared according to a method as described in WO 02/070483) were sequentially added to a 50 ml single-necked flask, and stirred at room temperature, until the reaction was completed as indicated by HPLC. The reaction solution was poured into water (50 ml), and extracted with dichloromethane (3 × 20 ml). The organic phase was washed sequentially with saturated sodium carbonate solution, saturated aqueous sodium chloride solution and water, and dried over anhydrous sodium sulfate. The solvent was removed under reduced pressure, and the resulting crude product was purified by column chromatography (eluent: ethyl acetate: petroleum ether = 3:1), to obtain a white solid (0.23 g, yield 41.2%).

### Example 2

### Synthesis of Compound 1.3: N-methyl-N-(2-N-((1-amino-2-methyl-1-acylpropan-2-yl)-formyl)-4-chloro-6-methyl phenyl)-1-(3-chloropyridin-2-yl)-3-bromo -1H-pyrazol-5-carboxamide

### (1) Synthesis of 2-methyl-2-(methylamino)propionitrile

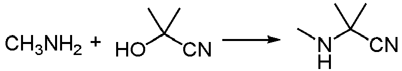

Acetone cyanohydrin (8.5 g, 0.1 mol) was added to a 50 ml four-necked flask, and then methylamine gas (3.1 g, 0.1 mol) was slowly introduced at room temperature. After that, the reaction was continuously stirred at room temperature for 5 h, and extracted with dichloromethane (10 ml × 3). The organic phase were combined and dried over anhydrous sodium sulfate. Then, the solvent was removed under reduced pressure to obtain a colorless transparent liquid (6.18 g, yield 63.0%).

### (2) Synthesis of 2-nitro-3-methylbenzoyl chloride

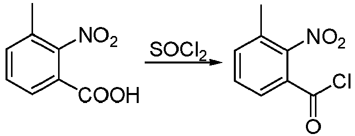

2-nitro-3-methylbenzoic acid (5.4 g, 0.03 mol), dichloroethane (100 ml), chlorosulfoxide (23.8 g, 0.2 mol), and DMF (1 drop) were sequentially added to a 250 ml single-necked flask, heated to reflux and reacted for 3 h. Then, the solvent was removed under elevated pressure to obtain a brown liquid (5.7 g, yield 95.2%). The product was directly used in next step without further post-treatment.

### (3) Synthesis of N-(1-cyanoisopropyl)-N-methyl-3-methyl-2-nitrobenzamide

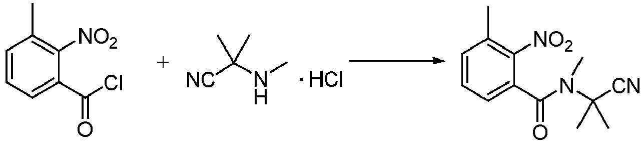

2-methyl-2-(methylamino)propionitrile hydrochloride (4.0 g, 0.03 mol), tetrahydrofuran (10 ml), water (10 ml), and NaHCOs (5.1 g, 0.06 mol) were sequentially added to a 250 ml four-necked flask. 2-nitro-3-methylbenzoyl chloride (5.7 g) in tetrahydrofuran (20 ml) was added dropwise at -10°C, and then continuously reacted for 2 h with stirring at -10°C. The reaction solution was added with water (50 ml), and extracted with ethyl acetate (20 ml × 3). The organic phases were combined, washed with saturated aqueous sodium chloride solution, and dried over anhydrous sodium sulfate. The solvent was removed under reduced pressure to obtain a brown solid (4.4 g, yield 56.5%).

### (4) Synthesis of N-(1-cyanoisopropyl)-N-methyl-3-methyl-2-aminobenzamide

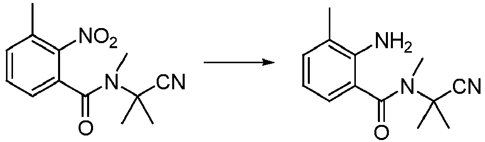

Water (20 ml), reduced Fe powder (2.2 g, 0.04 mol), and 30% hydrochloric acid (1 ml) were sequentially added to a 100 ml four-necked flask, slowly heated to 80°C and stirred for 30 min at 80°C. Then, N-(1-cyanoisopropyl)-N-methyl-3-methyl-2-nitrobenzamide (2.6 g, 0.01 mol) was added batchwise, while the temperature was maintained at no more than 80°C. After that, the reaction was continuously stirred at 80°C, until the reaction was completed as indicated by HPLC. The reaction solution was cooled to room temperature, added with sodium hydroxide (1.6 g), and filtered with suction. The filter cake was washed with hot water, and the collected filtrate was extracted with ethyl acetate (2 × 100 ml). The organic phase was washed with water, saturated sodium carbonate solution and saturated brine, and dried over anhydrous sodium sulfate. The solvent was removed under reduced pressure to obtain a brown solid (1.68 g, yield 71.3%).

### (5) Synthesis of N-(1-cyanoisopropyl)-N-methyl-2-amino-3-methyl-5-chlorobenzamide

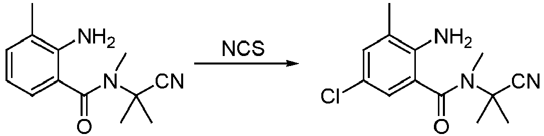

N-(1-cyanoisopropyl)-N-methyl-3-methyl-2-aminobenzamide (1.6 g, 6.9 mmol), N-chlorosuccinimide (1.4 g, 10.3 mmol) and DMF (20 ml) were sequentially added to a 100 ml four-necked flask, and reacted with stirring at room temperature until the reaction was completed as indicated by HPLC. The reaction solution was poured into water (100 ml), and extracted with ethyl acetate (20 ml × 3). The organic phases were combined, washed sequentially with saturated aqueous sodium chloride solution and water, and dried over anhydrous sodium sulfate. The solvent was removed under reduced pressure to obtain a light yellow solid (1.52 g, yield 83.1%).

### (6) Synthesis of N-(1-amino-2-methyl-1-acylpropan-2-yl)-N-methyl-2-amino-3-methyl-5-chlorobenza mide

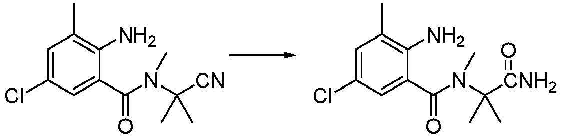

In an ice bath, N-(1-cyanoisopropyl)-N-methyl-2-amino-3-methyl-5-chlorobenzamide (0.80 g, 3.0 mmol), tetrahydrofuran (10 ml), potassium hydroxide (0.35 g, 6.0 mmol) in water (5 ml), and 30% hydrogen peroxide solution (2 g, 18 mmol) were sequentially added to a 100 ml single-necked flask, naturally warmed to room temperature, and reacted with stirring, until the reaction was completed as indicated by HPLC. The solvent was removed from the reaction solution under reduced pressure to obtain a brownish-yellow solid. The solid was washed with tetrahydrofuran (10 ml × 2), and filtered. The filtrate was collected, and removed of the solvent under reduced pressure. The crude product was purified column chromatography (petroleum ether: ethyl acetate = 1:1) to obtain a white solid (0.31 g, yield 36.7%).

### (7) Synthesis of N-methyl-N-(2-N-((1-amino-2-methyl-1-acylpropan-2-yl)-formyl)-4-chloro-6-methyl phenyl)-1-(3-chloropyridin-2-yl)-3-bromo-1H-pyrazol-5-carboxamide

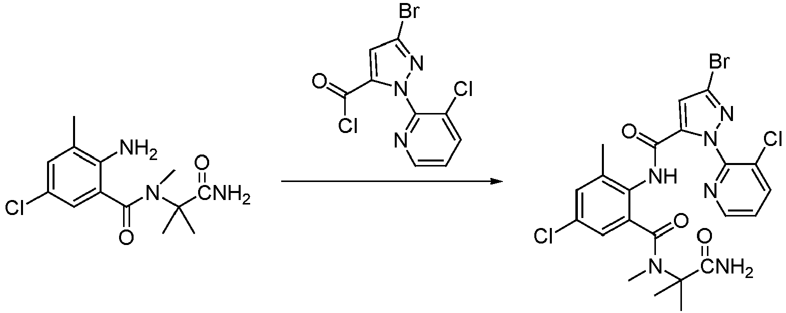

N-(1-amino-2-methyl-1-acylpropan-2-yl)-N-methyl-2-amino-3 -methyl-5-chlorob enzamide (0.30 g, 1 mmol), acetonitrile (10 ml), pyridine (0.079 g, 1 mmol), and 2-(3-chloro-pyridin-2-yl)-5-bromo-2H-pyrazol-3-carbonyl chloride (0.32 g, 1 mmol) (prepared according to a method as described in WO 02/070483) were sequentially added to a 50 ml single-necked flask, and stirred at room temperature until the reaction was completed as indicated by HPLC. The reaction solution was poured into water (50 ml), and extracted with dichloromethane (3 × 20 ml). The organic phase was washed sequentially with saturated sodium carbonate solution, saturated aqueous sodium chloride solution and water, and dried over anhydrous sodium sulfate. The solvent was removed under reduced pressure, and the resulting crude product was purified by column chromatography (eluent: ethyl acetate: petroleum ether = 3:1), to obtain a white solid (0.24 g, yield 42.8%).

The other compounds could be synthesized by the above method.

### Biological activity test

The test reagents were all individually dissolved in a mixed solvent of acetone:N'N'-dimethylcarboxamide (1:1) to give a 1000 mg/L solution. 1% Tween-80 was added as an emulsifier to each solution. These solutions were then diluted with 1% Tween-20 solution in water to give desired concentrations of test solutions. An aqueous solution containing 1% Tween-20 was used as a control.

### Example 3: Insecticidal effect on Spodoptera litura

3 rd instar larvae of *Spodoptera litura* were used, and the insecticidal effect test was carried out by feeding with leaves immersed with various compounds. The cabbage leaves that had not been exposed to insecticides were cut into leaf discs of about 40 square-mm with scissors. The leaf discs were immersed in each compound solution for 30s. Then the leaf discs were placed on absorbent paper and air-dried until there were no obvious water stains on the leaf discs. The leaf discs soaked with the reagents were placed in a petri dish (7 cm), each petri dish having 3 leaf discs. The 3rd instar larvae of *Spodoptera litura* raised on the indoor cabbage plants were gently picked up with a pen brush and placed on the leaf discs in the petri dish, each petri dish having 10-15 insects. After the insects were inoculated, the petri dish was covered, and placed in an insect-cultivating chamber at 25°C with a 16-h-light/8-h-dark photoperiod. The number of deaths in 5 days was investigated, and the mortality was calculated.

When the concentration of the reagent solution is 40 ppm, some compounds such as 1.1 have a better control effect on *Spodoptera litura,* reaching more than 80%.

### Example 4: Insecticidal effect on Jacobiascaformosana

The leaves (with petioles) on peach tree were cut, and more than 10 2nd-to-4th-instar nymphs (old nymphs were removed with sharp needles) of *Jacobiasca formosana* were present on the leaves. The leaves with the nymphs were immersed in the test solutions of each reagent for 20s, and then taken out. The petiole was wrapped with absorbent cotton. The leaves were laterally placed in a Petri dish with filter paper, and the water on the surface of the leaves was absorbed by the filter paper. Then the Petri dish was covered. 3 replicates were set for each treatment. After the treatment, the petri dish was placed in an insect cultivating incubator at 25°C light with a 16-h-light/8-h-dark photoperiod. 72 hrs after the insects were inoculated, the deaths of *Jacobiasca formosana* were investigated to calculate the mortality.

When the concentration of the reagent solution is 40 ppm, some compounds such as 1.1 and 1.3 have a better control effect on *Jacobiasca formosana,* reaching more than 80%.

### Example 5: Insecticidal effect on Frankliniella intonsa

On the bottom of a glass petri dish with a diameter of 6 cm, a piece of filter paper with the same diameter was laid. 1 mL of the reagent solution was drawn by a pipette and uniformly dripped on the filter paper. After the acetone was volatilized, Nymphs of *Frankliniella intonsa* were transferred from *Bombax ceiba* with a brush pen to the filter paper in the petri dish. After 0.5-1 h when the insect body was fully covered with the reagent, 3 cm-long petals of *Bombax ceiba* were placed and the Petri dish were covered. 3 replicates were set for each treatment. After the insects were inoculated, the petri dish was placed in an insect-cultivating chamber at 25°C with a 16-h-light/8-h-dark photoperiod. 72 hrs after the insects were inoculated, the deaths of *Frankliniella intonsa* were investigated to calculate the mortality.

When the concentration of the reagent solution is 60 ppm, some compounds such as 1.1 have a better control effect on *Frankliniella intonsa,* reaching more than 80%.

### Example 6: Insecticidal effect on Nilaparvata lugens

Soil was washed off from the root of 60-day-old seedlings and the leaves were cut off. After air drying, the remaining portion was immersed for 30s in the test solution of each reagent, then taken out, placed on absorbent paper and air-dried. The root crown was wrapped with absorbent cotton, and the root was immersed in clear water through the bottle cap. The above-ground portion was housed in a plastic insect cultivating bottle (with 3 × 2 cm square holes on both sides and sealed with a fine mesh); and placed in a plastic cup. 3 replicates were set for each treatment. 3rd to 4th instar nymphs of *Nilaparvata lugens* raised on indoor rice seedlings were gently picked up with a brush pen and placed on seedlings in plastic cups, and 10 insects per cup were inoculated. After the insects were inoculated, the plastic cup was placed in an insect-cultivating chamber at 25°C with a 16-h-light/8-h-dark photoperiod. 120 hrs after the insects were inoculated, the deaths of *Nilaparvata lugens* were investigated to calculate the mortality.

When the concentration of the reagent solution is 40 ppm, some compounds such as 1.1 and 1.2 have a better control effect on *Nilaparvata lugens,* reaching more than 80%.

### Example 7: Insecticidal effect on Helicoverpa armigera

3rd instar larvae of *Helicoverpa armigera* were used, and the insecticidal effect test was carried out by feeding with leaves immersed with various compounds. The cabbage leaves that had not been exposed to insecticides were punched into leaf discs having a diameter of 1.5 mm. The leaf discs were immersed in each compound solution for 30s. Then the leaf discs were placed on absorbent paper and air-dried until there were no obvious water stains on the leaf discs. A washed 24-well plate was prepared, and about 1 ml of water agar medium was fed to each well. After solidification, the treated leaf discs were placed on the medium in the 24-well plate, and then 1 3rd-instar larva of *Helicoverpa armigera* was inoculated into each well. The 24-well plate was covered after inoculation, placed in an insect cultivating chamber at 25°C with a 16-h-light/8-h-dark photoperiod. The number of deaths in 3 days was investigated, and the mortality was calculated.

Some test results are as follows:
When the concentration of the reagent solution is 40 ppm, the mortality of 3rd instar larvae of *Helicoverpa armigera* caused by some compounds such as 1.1, 1.2, and 1.3 is 80% or higher.

According to the above method, Comparative Compounds KC1 (Compound 1.3 in CN 104447688) and KC2 (Compound 1.4 in CN 104447688) in the prior art which are closest in structure to the compound of the present invention were tested for the insecticidal activity on *Helicoverpa armigera.* The experimental results are shown in Table 4 below.

Parallel comparison of the insecticidal activity of Compounds 1.1 and 1.2 of the present invention with known Compound KC1 and KC2 on *Helicoverpa armigera* (mortality%)

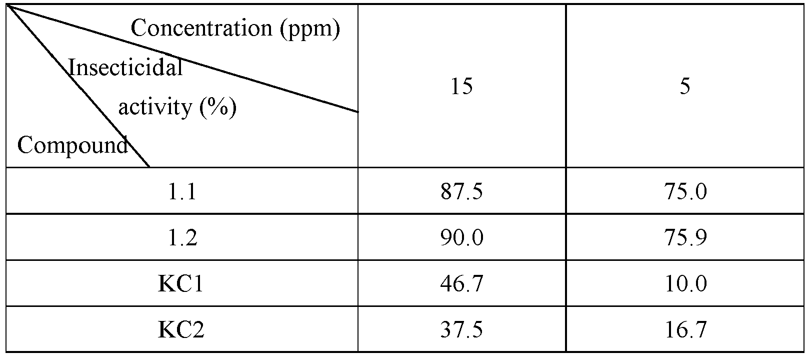

It can be seen from the above table that when used in the killing of *Helicoverpa armigera,* compared with Compound KC1 and Compound KC2 disclosed in the prior art, the killing rate of the compound of the present invention for *Helicoverpa armigera* can still reach 70% at a concentration of less than 20 ppm, even at a concentration of 5 ppm. Therefore, compared with Compound KC1 and KC2 in the prior art, the activity of the compound of the present invention is much higher.

## Claims

1. A pyrazolamide compound of General Formula I,: 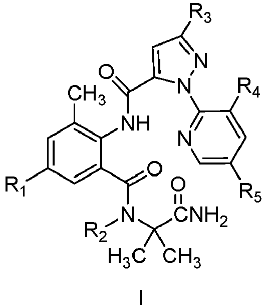 wherein
R₁ is selected from chloro or CN;
R₂ is selected from H, C₁-C₃alkyl, C₁-C₃ haloalkyl or C₁-C₅ alkoxyalkyl;
R₃ is selected from halo or C₁-C₃ haloalkyl;
R₄ is selected from halo; and
R₅ is selected from H or halo.

2. The pyrazolamide compound having an insecticidal activity according to claim 1, wherein in preferred compounds of General Formula I:
R₁ is selected from chloro or CN;
R₂ is selected from H, C₁-C₃alkyl, CH₂OCH₃, CH₂OCH₂CH₃, CH₂CH₂OCH₃, CF₃, CH₂CH₂F, CH₂CHF₂, CH₂CF₃, CF₂CF₃ or CF(CF₃)₂;
R₃ is selected from chloro, bromo or trifluoromethyl;
R₄ is selected from chloro; and
R₅ is selected from H or chloro.

3. The pyrazolamide compound having an insecticidal activity according to claim 1, wherein in preferred compounds of General Formula I:
R₁ is selected from chloro;
R₂ is selected from H or methyl;
R₃ is selected from chloro or bromo;
R₄ is selected from chloro; and
R₅ is selected from H.

4. A compound, having a structure of General Formula II: 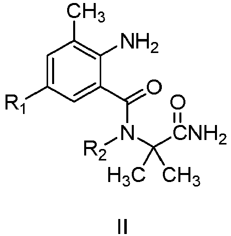 wherein
R₁ is selected from chloro or CN; and
R₂ is selected from H, C₁-C₃alkyl, C₁-C₃ haloalkyl or C₁-C₅ alkoxyalkyl.

5. A method for preparing a compound of General Formula I according to claim 1, comprising:
reacting a compound of General Formula II with a compound of General Formula III to obtain a compound of General Formula I, wherein the reaction scheme is shown below: 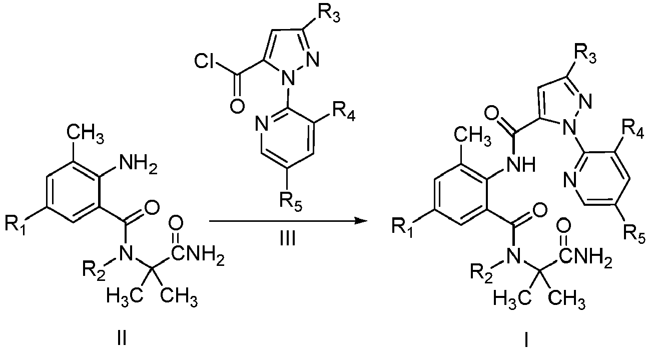 wherein
R₁ is selected from chloro or CN;
R₂ is selected from H, C₁-C₃ alkyl, C₁-C₃ haloalkyl or C₁-C₅ alkoxyalkyl;
R₃ is selected from halo or C₁-C₃ haloalkyl;
R₄ is selected from halo; and
R₅ is selected from H or halo.

6. Use of the compound of General Formula I according to claim 1, 2 or 3 in the preparation of insecticides for controlling pests.

7. An insecticidal composition, comprising a compound of General Formula I according to claim 1, and an agriculturally, forestrically, and hygienically acceptable carrier, wherein the content in percentage by weight of the active ingredient in the composition is 0.1-99.5%.

8. A method for controlling pests, comprising applying an insecticidal composition according to claim 7 to the pests or their growth media excluding the human and animal body in an effective amount of 10 g/ hm²-1000g/ hm².

## Patentansprüche

1. Pyrazolamidverbindung der allgemeinen Formel I,: 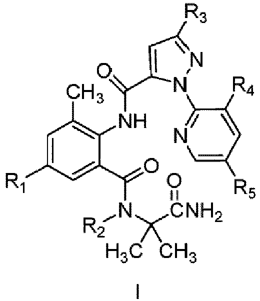 wobei
R₁ ausgewählt ist aus Chlor oder CN;
R₂ ausgewählt ist aus H, C₁-C₃-Alkyl, C₁-C₃-Haloalkyl oder C₁-C₅-Alkoxyalkyl;
R₃ ausgewählt ist aus Halo oder C₁-C₃-Haloalkyl;
R₄ ausgewählt ist aus Halo; und
R₅ ausgewählt ist aus H oder Halo.

2. Pyrazolamidverbindung mit insektizider Aktivität nach Anspruch 1, wobei bei bevorzugten Verbindungen der allgemeinen Formel I:
R₁ ausgewählt ist aus Chlor oder CN;
R₂ ausgewählt ist aus H, C₁-C₃-Alkyl, CH₂OCH₃, CH₂OCH₂CH₃, CH₂CH₂OCH₃, CF₃, CH₂CH₂F, CH₂CHF₂, CH₂CF₃, CF₂CF₃ oder CF(CF₃)₂;
R₃ ausgewählt ist aus Chlor, Brom oder Trifluormethyl,
R₄ ausgewählt ist aus Chlor; und
R₅ ausgewählt ist aus H oder Chlor.

3. Pyrazolamidverbindung mit insektizider Aktivität nach Anspruch 1, wobei bei bevorzugten Verbindungen der allgemeinen Formel I:
R₁ ausgewählt ist aus Chlor;
R₂ ausgewählt ist aus H oder Methyl;
R₃ ausgewählt ist aus Chlor oder Brom;
R₄ ausgewählt ist aus H.

4. Verbindung mit einer Struktur der allgemeinen Formel II: 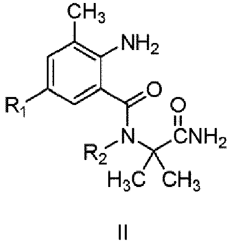 wobei
R₁ ausgewählt ist aus Chlor oder CN;
R₂ ausgewählt ist aus H, C₁-C₃-Alkyl, C₁-C₃-Haloalkyl oder C₁-C₅-Alkoxyalkyl.

5. Verfahren zur Herstellung einer Verbindung der allgemeinen Formel I nach Anspruch 1, umfassend:
Umsetzen einer Verbindung der allgemeinen Formel II mit einer Verbindung der allgemeinen Formel III, um eine Verbindung der allgemeinen Formel IV zu erhalten, wobei das Reaktionsschema untenstehend gezeigt ist: 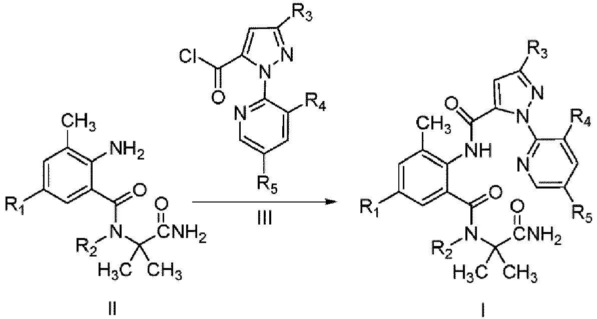 wobei
R₁ ausgewählt ist aus Chlor oder CN;
R₂ ausgewählt ist aus H, C₁-C₃-Alkyl, C₁-C₃-Haloalkyl oder C₁-C₅-Alkoxyalkyl;
R₃ ausgewählt ist aus Halo oder C₁-C₃-Haloalkyl;
R₄ ausgewählt ist aus Halo; und
R₅ ausgewählt ist aus H oder Halo.

6. Verwendung der Verbindung der allgemeinen Formel I nach Anspruch 1, 2 oder 3 bei der Herstellung von Insektiziden zur Schädlingsbekämpfung.

7. Insektizidzusammensetzung, aufweisend eine Verbindung der allgemeinen Formel I nach Anspruch 1 und einen landwirtschaftlich, forstwirtschaftlich und hygienisch verträglichen Träger, wobei der Anteil des Wirkstoffs in Gewichtsprozent in der Zusammensetzung 0,1 - 99,5 % beträgt.

8. Verfahren zur Schädlingsbekämpfung, umfassend das Auftragen einer Insektizidzusammensetzung nach Anspruch 7 auf Schädlinge oder deren Wachstumsmedium, mit Ausnahme des menschlichen und tierischen Körpers, in einer wirksamen Menge von 10g/hm² - 1000g/hm².

## Revendications

1. Composé pyrazolamide de formule générale I : 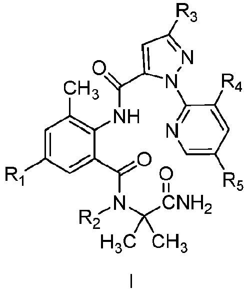 dans lequel
R₁ est choisi parmi chloro ou CN ;
R₂ est choisi parmi H, alkyle en C₁-C₃, haloalkyle en C₁-C₃ ou alcoxyalkyle en C₁-C₅ ;
R₃ est choisi parmi halo ou haloalkyle en C₁-C₃ ;
R₄ est choisi parmi halo ; et
R₅ est choisi parmi H ou halo.

2. Composé pyrazolamide ayant une activité insecticide selon la revendication 1, dans lequel dans les composés préférés de formule générale I :
R₁ est choisi parmi chlore ou CN ;
R₂ est choisi parmi H, alkyle en C₁-C₃, CH₂OCH₃, CH₂OCH₂CH₃, CH₂CH₂OCH₃, CF₃, CH₂CH₂F, CH₂CHF₂, CH₂CF₃, CF₂CF₃ ou CF(CF₃)₂ ;
R₃ est choisi parmi chloro, bromo ou trifluorométhyle ;
R₄ est choisi parmi chloro ; et
R₅ est choisi parmi H ou chloro.

3. Composé pyrazolamide ayant une activité insecticide selon la revendication 1, dans lequel dans les composés préférés de formule générale I :
R₁ est choisi parmi chloro ;
R₂ est choisi parmi H ou méthyle ;
R₃ est choisi parmi chloro ou bromo ;
R₄ est choisi parmi chloro ; et
R₅ est choisi parmi H.

4. Composé, ayant une structure de formule générale II : 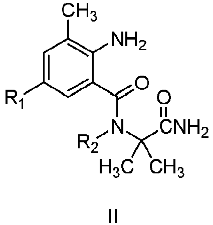 dans lequel
R₁ est choisi parmi chloro ou CN ; et
R₂ est choisi parmi H, alkyle en C₁-C₃, haloalkyle en C₁-C₃ ou alcoxyalkyle C₁-C₅.

5. Procédé de préparation d'un composé de formule générale I selon la revendication 1, comprenant :
la réaction d'un composé de formule générale II avec un composé de formule générale III pour obtenir un composé de formule générale I, dans lequel le schéma réactionnel est présenté ci-dessous : 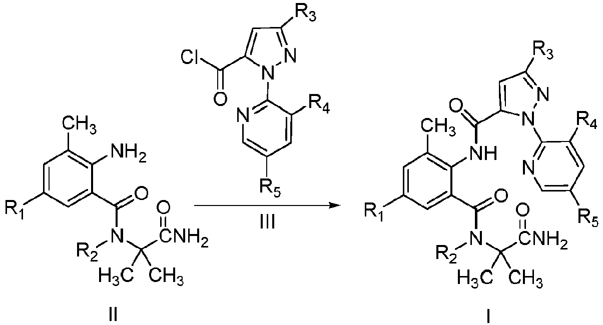 dans lequel
R₁ est choisi parmi chloro ou CN ;
R₂ est choisi parmi H, alkyle en C₁-C₃, haloalkyle en C₁-C₃ ou alcoxyalkyle en C₁-C₅ ;
R₃ est choisi parmi halo ou haloalkyle en C₁-C₃ ;
R₄ est choisi parmi halo ; et
R₅ est choisi parmi H ou halo.

6. Utilisation du composé de formule générale I selon la revendication 1, 2 ou 3 dans la préparation d'insecticides pour lutter contre des parasites.

7. Composition insecticide, comprenant un composé de formule générale I selon la revendication 1, et un véhicule acceptable du point de vue agricole, forestier et hygiénique, dans laquelle la teneur en pourcentage en poids de l'ingrédient actif dans la composition est de 0,1 à 99,5 %.

8. Procédé de lutte contre des parasites, comprenant l'application d'une composition insecticide selon la revendication 7 aux parasites ou à leurs milieux de croissance, à l'exclusion du corps humain et animal, en une quantité efficace de 10 g/hm² à 1 000 g/hm².
